# FASCICULE DE BREVET EUROPEEN

(11) **EP 3 732 283 B1**
(45) Date de publication et mention de la délivrance du brevet: **24.11.2021**
(21) Numéro de dépôt: 18829412.8
(22) Date de dépôt: 26.12.2018
(51) Int. Cl.: C12M 1/12, C12M 1/06, C12M 3/00, C12M 1/34, C12M 1/00

(54) **DISPOSITIF DE CULTURE DE MICRO-ORGANISMES**
VORRICHTUNG ZUR KULTIVIERUNG VON MIKROORGANISMEN
DEVICE FOR CULTIVATING MICROORGANISMS

(30) Priorité: 26.12.2017 FR 1763222
(43) Date de publication de la demande: 04.11.2020
(73) Titulaire: Centre National de la Recherche Scientifique (CNRS), 75016 Paris (FR); Pierre Guerin, 79210 Mauzé-sur-le Mignon (FR)
(72) Inventeur: OUAZZANI CHAHDI, Jamal-Eddine, 91300 MASSY (FR); LE GOFF, Géraldine, 92160 ANTONY (FR); FELEZEU, Doru, 75013 PARIS (FR); TOURON, Arnaud, 79360 GRANZAY-GRIPT (FR); ALLEGRET-BOURDON, Christophe, 79210 LE BOURDET (FR)
(74) Mandataire: Regimbeau
(86) Numéro de dépôt international: PCT/EP2018/086882
(87) Numéro de publication internationale: WO 2019/129787

(56) Documents cités:
- FR-A1- 2 586 426
- FR-A1- 2 901 280
- DATABASE WPI Week 201033 Thomson Scientific, London, GB; AN 2010-D16612 XP002784685, -& CN 101 665 764 A (XU Y) 10 mars 2010 (2010-03-10)

## Description

### DOMAINE TECHNIQUE GENERAL ET ETAT DE LA TECHNIQUE

La présente invention concerne le domaine de la culture de cellules. Elle s'applique en particulier à la culture de micro-organismes. Cependant, elle n'est pas limitée à cette application particulière et s'étend à la culture de tout type cellulaire (cellules végétales, cellules d'insectes).

La recherche et le développement de molécules d'intérêts, à partir de substances naturelles, produites par diverses bio-ressources et notamment par les microorganismes, constituent un enjeu majeur dans le domaine thérapeutique, cosmétique et phytosanitaire.

Cette recherche nécessite des équipements permettant la culture de micro-organismes dans des conditions favorables à la production des molécules d'intérêt. FR 2 901 280 divulgue un dispositif de culture, comprenant une enceinte (100) constituée d'une cuve (110) et d'un couvercle (150), adaptée pour définir à l'état fermé un volume étanche sous pression contrôlée, et un ensemble (200) conçu pour être placé à l'intérieur de l'enceinte (100) comprenant une pluralité de plateaux (250) portés, l'enceinte (100) comprenant par ailleurs des moyens (113, 160, 130, 114) de contrôle de l'atmosphère de l'enceinte (100).

### PRESENTATION DE L'INVENTION

L'invention concerne un dispositif destiné à la culture de cellules ou micro-organismes qui comprend les éléments permettant aussi bien la culture liquide que solide et assure la possibilité de réaliser un suivi de la culture en fonction du temps (cinétique de production de biomolécules par le microorganisme cultivé).

A ce titre, l'invention concerne un dispositif destiné à la culture de cellules ou micro-organismes, comprenant une cuve et un couvercle définissant à l'état fermé une enceinte stérilisable et à atmosphère contrôlée, la cuve comprenant une chambre supérieure et une chambre inférieure en communication, le dispositif comprenant un ensemble configuré pour être placé à l'intérieur de la cuve dans la première chambre tout en étant susceptible d'être retiré de celle-ci, l'ensemble comprenant au moins un plateau destiné à recevoir un milieu de culture solide ou semi-solide de type gélose nutritive,

Le dispositif étant caractérisé en ce que la chambre inférieure est adaptée pour recevoir un milieu de culture liquide et en ce que le dispositif comprend en outre un système de contrôle du débordement du milieu liquide de la chambre inférieure vers la chambre supérieure afin d'éviter un transfert de matière de la chambre inférieure vers la chambre supérieure.

L'invention est avantageusement complétée par les caractéristiques suivantes, prises seules ou en une quelconque de leur combinaison techniquement possible :
- le dispositif comprend en outre un premier système de prélèvement d'un échantillon du milieu solide disposé dans la partie haute de la chambre supérieure, ledit premier système de prélèvement étant configuré pour prélever, de manière stérile, un échantillon du milieu solide sans perturber l'atmosphère régnant dans l'enceinte ;
- le premier système de prélèvement d'un échantillon comprend une pelle, de préférence, faisant partie d'un système fixe stérilisable in-situ situé en haut de la chambre supérieure qui permet de récupérer le contenu de la pelle sans perturber l'atmosphère régnant dans l'enceinte ;
- le dispositif comprend un deuxième système de prélèvement d'un échantillon du milieu liquide disposé dans la partie basse de la cuve au niveau de la chambre inférieure, ledit deuxième système de prélèvement étant configuré pour prélever un échantillon de milieu liquide sans perturber l'atmosphère régnant dans l'enceinte ;
- le système de contrôle du débordement est constitué d'un capteur de niveau du milieu liquide disposé dans la partie supérieure de la chambre inférieure et d'une arrivée de liquide anti-mousse disposée également dans la partie supérieure de la chambre inférieure ;
- la cuve comprend, au niveau de la chambre supérieure et de la chambre inférieure au moins une entrée permettant d'injecter un fluide ou un gaz ;
- la cuve comprend une double enveloppe à l'extérieur permettant, lorsqu'elle est alimentée au moyen d'un fluide ayant une température contrôlée, de stériliser et de contrôler de manière homogène la température interne de l'enceinte ;
- le dispositif comprend un axe central s'étendant depuis le haut de la cuve dans la chambre supérieure et un châssis support, l'ensemble d'au moins un plateau étant porté par ledit châssis support, ledit châssis étant configuré pour être disposé autour de l'axe, l'axe autorisant une rotation des plateaux ;
- le dispositif comporte en bas de la chambre inférieure une arrivée de gaz permettant d'une part le développement optimal des microorganismes et d'autre part le maintien de l'enceinte en surpression évitant ainsi toute contamination provenant de l'extérieure de l'enceinte.

La chambre supérieure et la chambre inférieure présentent les mêmes conditions atmosphériques. Ceci permet de cultiver les microorganismes dans des conditions physicochimiques similaires et de comparer ainsi l'efficacité de chacune des conditions de culture.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre une vue d'ensemble d'un dispositif selon l'invention ;
- la figure 2 illustre une vue schématique d'un dispositif selon l'invention ;

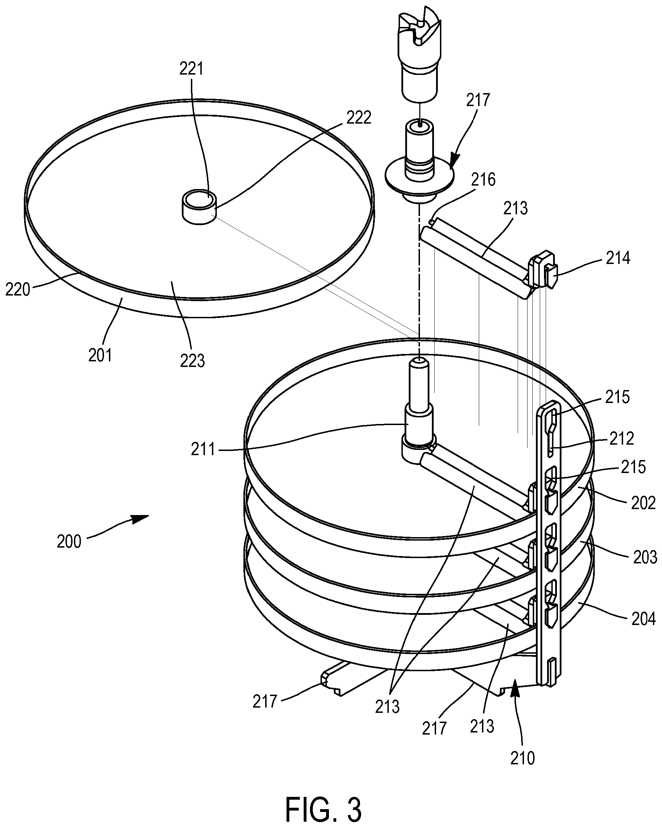

- la figure 3 illustre une vue d'une ensemble comprenant des plateaux destinés à recevoir un milieu de culture solide ou semi-solide dans un dispositif selon l'invention ;

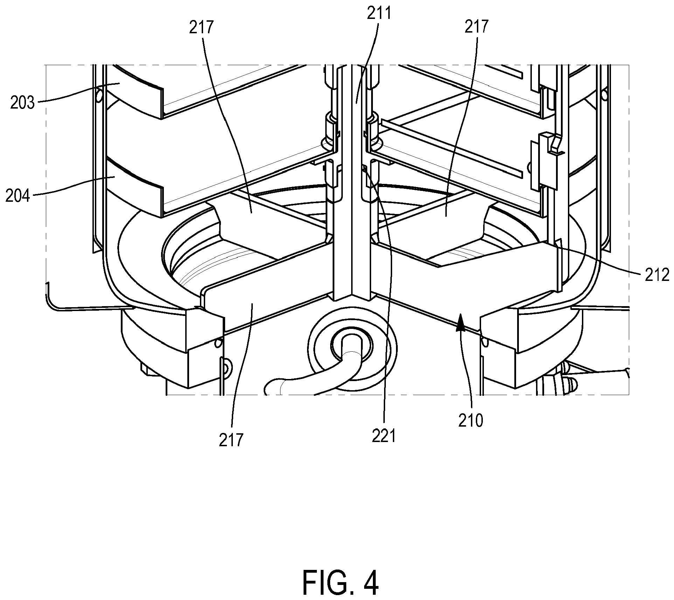

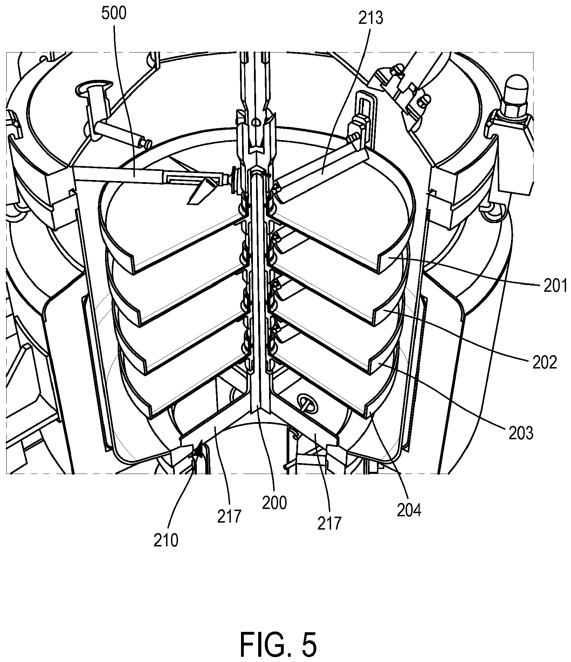

- les figures 4 et 5 illustrent des vues de l'intérieur d'un dispositif selon l'invention ;

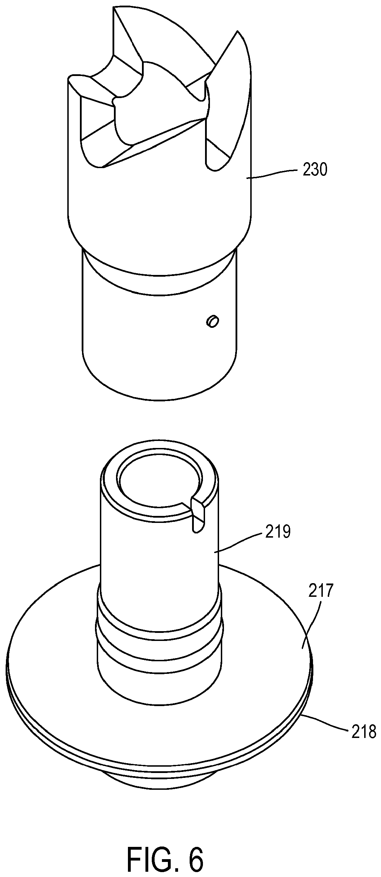

- La figure 6 illustre une vue d'une pièce d'entrainement d'un dispositif selon l'invention ;

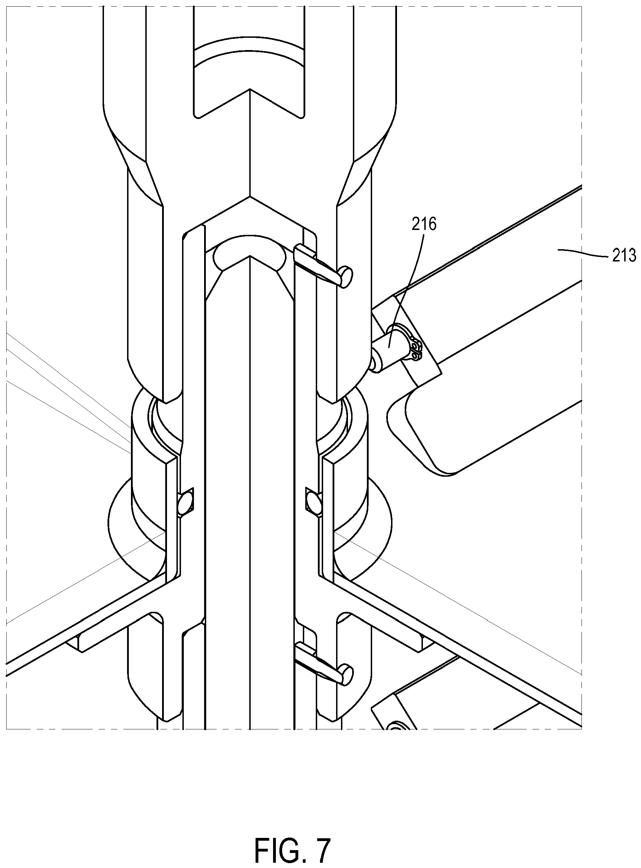

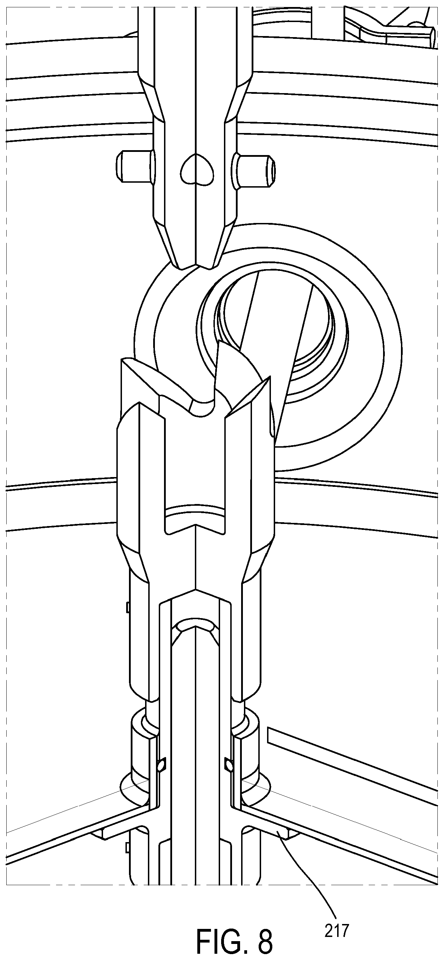

- Les figures 7 et 8 illustrent d'autres vues de l'intérieur d'un dispositif selon l'invention ;

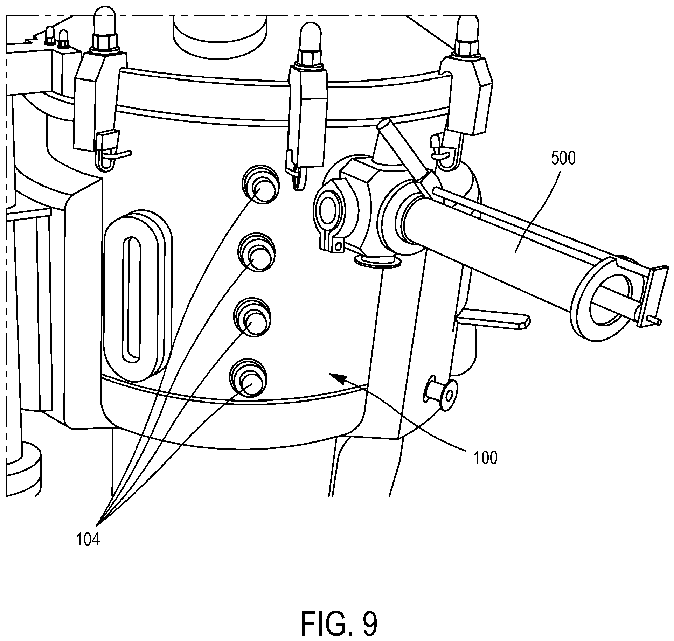

- La figure 9 illustre une vue d'un côté d'un dispositif selon l'invention ;

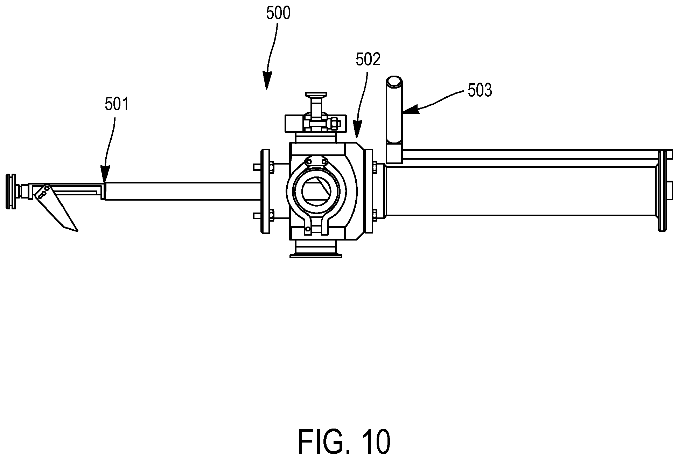

- La figure 10 illustre un dispositif de prélèvement stérile de milieu solide d'un dispositif selon l'invention.

Sur l'ensemble des figures, les éléments similaires portent des références identiques.

### DESCRIPTION DETAILLEE DE L'INVENTION

Un dispositif culture de cellules ou micro-organismes comprend une cuve 100 présentant une symétrie générale de révolution autour d'un axe vertical AA.

La cuve 100 est constituée d'une paroi 110 (et de préférence de deux parois reliées entre elles) et comprend une chambre supérieure 120 et une chambre inférieure 130 en communication l'une avec l'autre.

La cuve 100 est destinée à être maintenue sur un support et comprend à cet effet des attaches 101, 102 permettant d'attacher la cuve 100 à un support (non représenté). Les supports sont disposés de manière diamétralement opposée autour de la cuve au niveau de la chambre supérieure 120.

La hauteur interne de la cuve 100 est comprise entre 1100 et 1300 mm, typiquement 1274 mm.

Un couvercle 103 surmonte la cuve 100 et permet de définir une enceinte hermétiquement close à l'intérieur de laquelle règne une atmosphère contrôlée.

La chambre supérieure 120 est destinée à recevoir un ensemble 200 comprenant au moins un plateau 201, 202, 203, 204 destiné à recevoir un milieu de culture solide, organiques ou inorganiques, de préférence une gélose nutritive.

Avantageusement, les plateaux contiennent un milieu gélosé mais sont ensemencés avec des pré-cultures liquides de sorte que la culture sur les plateaux se fait sur milieu solide.

De préférence l'ensemble 200 est constitué d'un châssis 210 supportant un support 212 de pales 213. Les pales 213 sont destinées à répartir ou à homogénéiser le milieu disposé sur les plateaux. En outre, la position et la configuration des pales sont variables en fonction du milieu solide utilisé.

Le châssis peut prendre plusieurs formes. Il est de préférence composé d'une colonne 211 fixe verticale (le long de l'axe vertical AA du dispositif) autour duquel s'étendent les différents plateaux 201, 202, 203, 204 et d'un support horizontal constitué de quatre plaques 217 disposées radialement et régulièrement autour de la colonne 211.

Le couvercle 103 est formé d'une plaque et supporte, outre plusieurs accessoires nécessaires à la culture, un moteur 600 qui permet d'entrainer en rotation l'ensemble 200 (voir ci-après).

Chaque plateau 201, 202, 203, 204 est de préférence globalement cylindrique de révolution et comprend une base 223 plane circulaire surmontée à sa périphérie d'une jupe 220 cylindrique externe. La hauteur de cette jupe 220 externe peut être variable et est typiquement de l'ordre de 5 cm pour un diamètre de plateau de l'ordre de 50 cm. De manière non limitative, l'ensemble comprend quatre plateaux. Bien entendu, un nombre et une géométrie différents de plateaux peuvent être prévus et sont fonction du volume de la chambre supérieure.

Une telle structure de plateau 201, 202, 203, 204 est adaptée pour recevoir tout type de milieux de cultures mais préférentiellement une culture sur milieu de type gélose nutritive.

Les plateaux 201, 202, 203, 204 comprennent un orifice 221 central surmonté d'une jupe 222 cylindrique interne.

Les plateaux sont disposés le long de colonne fixe via leur orifice 221 central et repose chacun sur une embase centrale 217 constituée d'une base 218 plane surmontée d'une colonne 219 d'embase creuse. Ainsi, chaque embase de chaque plateau est insérée dans la colonne 211 fixe et est configurée pour coopérer avec l'embase du plateau au-dessus.

Au-dessus du dernier plateau 201 l'embase 217 correspondante est surmontée d'une pièce 230 d'entrainement destinée à coopérer avec un arbre 601 entrainé en rotation par le moteur 600 disposé au-dessus du dispositif sur le couvercle 103. L'embase 217 du haut est notamment entrainée en rotation par le moteur 600 et entraine par la même occasion les embases du dessous pour faire tourner les plateaux autour de la colonne 211 fixe verticale qui s'étend dans le dispositif le long de l'axe AA.

Les pales 213 sont disposées au niveau de chaque plateau 201, 202, 203, 204 pour permettre d'étaler la pré-culture dans chaque plateau 201, 202, 203, 204. Chaque pale 213 s'étend depuis le support de pales 213 disposé sur un des côtés du châssis 210 supportant l'axe fixe 211 (qui est une tige) et sont montées, chacune pivotante autour d'un barreau 216 destiné à être au-dessus d'un plateau. Les barreaux 216 sont suspendus au-dessus de chaque plateau de sorte que les pales restent fixes pendant la rotation des plateaux. En particulier, les pales 213 sont fixées au support 212 de pales par l'intermédiaire d'une pièce 214 encliquetée dans un logement 215 prévu dans le support de pales. Les pales 213 montées pivotantes sur les barreaux 216 permettent de pouvoir balayer la surface du milieu sans pénétrer celui-ci.

La chambre inférieure 130 est quant à elle destinée à recevoir un milieu de culture liquide.

Le volume de la chambre supérieure 120 est typiquement de 224 L. Le volume de la chambre inférieure 130 est typiquement de 66 L.

De manière avantageuse, le volume utile de la chambre inférieure 130 est de 40 L. Un tel volume utile est inférieur au volume de la chambre inférieure 130 pour éviter que le milieu liquide ne déborde pas sur un ou plusieurs plateau(x) 201, 202, 203, 204 (nous reviendrons plus loin sur cet aspect).

Le diamètre interne de la chambre supérieure 120 est compris entre 600 et 700 mm, typiquement 650 mm. Le diamètre interne de la chambre inférieure 130 est typiquement de 340 mm.

Avantageusement, la chambre supérieure 120 et la chambre inférieure 130 présentent les mêmes conditions atmosphériques.

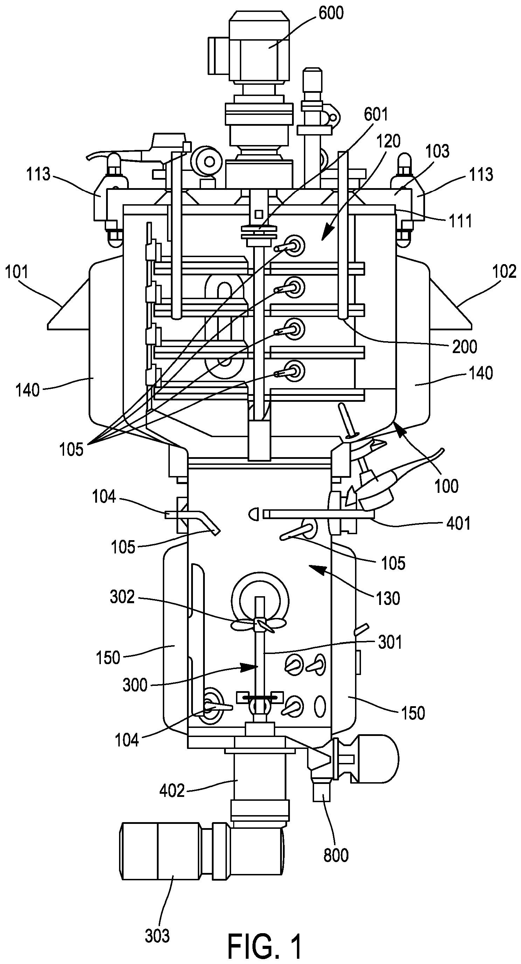

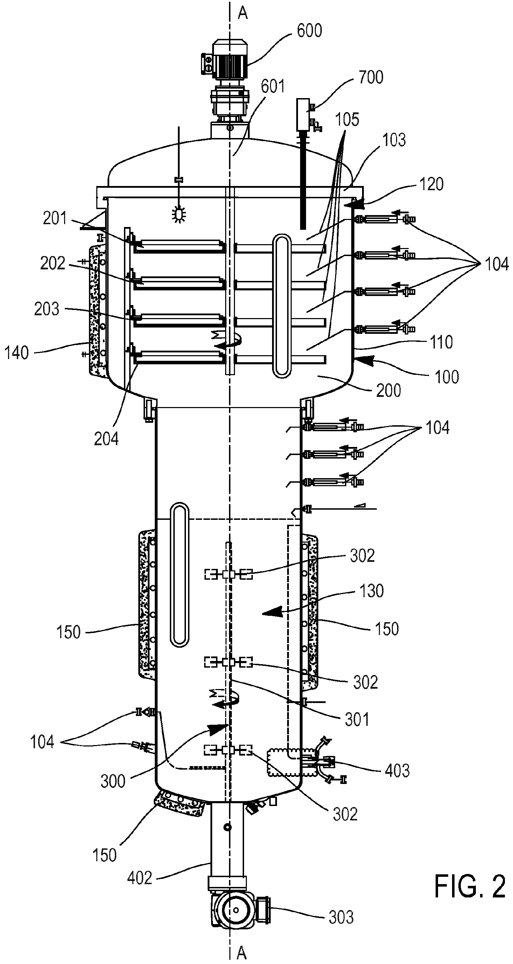

Les deux chambres 120, 130 sont cylindriques autour de l'axe vertical AA et peuvent présenter des dimensions différentes. Sur le dispositif de la figure 1, la chambre supérieure 120 présente un volume supérieur au volume de la chambre inférieure 130. Ainsi, comme cela est visible sur les figures 1 et 2, depuis la chambre supérieure 120 vers la chambre inférieure la cuve 100 présente un rétrécissement. Un tel rétrécissement permet d'avoir pour les deux chambres des géométries conformes aux dimensions usuelles dans le domaine, avec des plateaux de 50 cm de diamètres et une chambre 130 respectant un ratio diamètre/hauteur de 1/3.

La paroi externe de la cuve 100 comprend sur la quasi-totalité de la hauteur de chacune des chambres supérieure 120 et inférieure 130, une chambre annulaire supérieure 140 et une chambre annulaire inférieure 150 formant deux doubles enveloppes autour de chacune des chambres supérieure 120 et inférieure 130. Ces deux chambres annulaires 140, 150-sont alimentées en un fluide à température, qui permet de contrôler de manière homogène la température du volume interne de l'enceinte à l'intérieur de la cuve 100.

Les chambres annulaires 140, 150 autour de la chambre supérieure 120 et de la chambre inférieure 130 peuvent être formées de manière continues autour de la paroi externe de la cuve 100 ou bien constituées de manière indépendante.

Les chambres annulaires 140, 150 ne couvrent pas la totalité de la périphérie de la cuve 100 au niveau des chambres supérieure 120 et inférieure 130 et possèdent une section droite en C qui s'étend entre environ 300 à 320° autour de la paroi 110 de la cuve 100.

Le fait que les chambres annulaires 140, 450 ne couvrent pas la totalité de la cuve 100 permet de disposer des moyens nécessaires à la culture.

En particulier, au niveau de la chambre supérieure 120 et de la chambre inférieure 130 sont disposés des piquages 104 constitués par des ouvertures pratiquées dans la paroi de la cuve 100 et bouchées de manière à assurer l'étanchéité lorsque des tuyaux 105 traversant les bouchons sont retirés.

Dans la chambre supérieure 120, les tuyaux 105 permettent d'ensemencer des milieux de culture dans les plateaux 201, 202, 203, 204, de sorte qu'il est prévu autant de piquage que de plateaux 201, 202, 203, 204 susceptibles d'être introduits dans la cuve 100. De plus, les tuyaux 105 dans la chambre supérieure 120 sont disposés au-dessus de chaque plateau 201, 202, 203, 204. Les dispositifs de piquage 104 sont prévus pour être stérilisables et introduire des solutions épaisses contenant des mélanges complexes d'inoculum et de résines ou tout autre moyen de piégeage de biomolécules in situ (en anglais, « *Solid Phase Extraction* », (SPE)).

Dans la chambre inférieure 130, les tuyaux 105 permettent d'introduire le milieu liquide à cultiver. On note toutefois, que le milieu liquide peut être introduit par le haut de la cuve 100 avant la stérilisation de cette dernière et complémenté de tout moyen de piégeage de biomolécules in situ (en anglais, « Solid Phase Extraction », (SPE)).

De manière plus générale, les tuyaux 105 permettent d'introduire toutes sortes de produits nécessaires à la culture des différents milieux dans la cuve 100 et peuvent être de différentes tailles.

Les tuyaux 105 sont donc reliés à l'extérieur de la cuve 100 à diverses sources (non représentées) pour amener les milieux et/ou les différents produits.

Au fond de cuve 100 est disposé un agitateur 300 permettant d'agiter le milieu liquide dans la chambre inférieure 130. L'agitateur 300 est constitué d'une tige 301 supportant trois hélices 302 et entrainée par un moteur 303 électrique à l'extérieure de la cuve 100. Les hélices 302 sont réparties le long de la tige 301. Bien entendu, le nombre et la forme pour les hélices ne sont pas limitatifs.

Afin d'éviter un débordement depuis la chambre inférieure 130 vers la chambre supérieure 120 du milieu liquide, notamment lors de l'agitation de ce dernier, le dispositif comprend un système 401 de contrôle du débordement. Un tel système de contrôle du débordement comprend un capteur 401 de niveau du milieu liquide. Un débordement peut être constitué soit du milieu liquide soit d'une mousse formée par l'agitation du milieu liquide. Un tel débordement est préjudiciable car le milieu liquide peut porter atteinte aux cultures réalisées dans les plateaux 201, 202, 203, 204 au-dessus.

Le capteur 401 de niveau est en outre couplé au déclenchement de l'introduction d'un liquide anti-mousse au moyen de l'un des piquages 104 disposés au niveau de la chambre 130 inférieure.

Un tel produit est par exemple des tensioactifs naturels ou estérifiés.

Avantageusement, le dispositif comprend un premier système 500 de prélèvement d'un échantillon de milieu solide disposé sur la paroi de la cuve 100. Un tel premier système de prélèvement permet de prélever un échantillon du milieu solide sans dégrader l'atmosphère régnant dans l'enceinte. Un tel premier système de prélèvement est constitué d'une « mini-pelle » comprenant un tube 501 creux qui permet d'amener un échantillon prélevé par la pelle à l'extérieur de la cuve. Ce premier système est fixé de manière étanche à la cuve et est notamment configuré pour permettre le prélèvement d'un échantillon sans dégrader l'atmosphère qui règne à l'intérieur de la cuve 100. A ce titre, le premier système 500 de prélèvement comprend un ensemble 502 étanche comprenant un double sas qui permet de manière séquentielle d'extraire l'échantillon sans dégrader l'atmosphère régnant dans la cuve 100. La mini-pelle est manoeuvrable de manière manuelle par un opérateur au moyen d'un bras 503.

Pour des raisons pratiques, le premier système 500 de prélèvement est disposée sur le premier plateau en partant depuis le haut de la cuve. Il s'agit du seul plateau qui ne comprend pas un autre plateau au-dessus de lui.

De manière alternative ou complémentaire, le dispositif comprend un deuxième système 403 de prélèvement d'un échantillon du milieu liquide disposé au fond de la cuve 100 au niveau de la chambre inférieure 130. Là aussi, le deuxième système 403 de prélèvement est configuré pour prélever un échantillon de milieu liquide sans perturber l'atmosphère régnant dans l'enceinte. Un tel deuxième système, est constituée d'une prise 403 disposée au fond de la cuve 100. Une telle prise 403 permet de prélever un échantillon de liquide.

Les premier et deuxième systèmes ci-dessus décrits permettent de prélever à différents instants des échantillons afin de suivre la cinétique de production des produits d'intérêt dans l'enceinte dans la chambre supérieure 120 et/ou dans la chambre inférieure 130 le tout en ne dégradant pas l'atmosphère régnant dans l'enceinte.

Afin de supporter le couvercle 103, la cuve 100 comprend une bride 111 au niveau de la chambre supérieure 120. En outre, le dispositif comprend des moyens de verrouillage 112, 113 du couvercle 103 pour assurer le verrouillage du couvercle 103 de manière étanche toujours dans l'objectif d'avoir une atmosphère contrôlée dans l'enceinte. En particulier, un joint d'étanchéité (non représenté) est intercalé entre la bride 111 de la cuve 100 et la périphérie de la base du couvercle 103.

Le dispositif comprend également au moins une sonde 700 de température adaptée pour mesurer la température interne de l'enceinte. Une sonde de température est fixée sur la paroi 110 de la cuve 100 au niveau de la partie basse de la chambre supérieure 120 et l'autre est disposée sur le couvercle 103.
De manière non limitative les chambres 120 et 130 peuvent être équipées de différents moyens de mesure et de contrôle de l'agitation, de la pression, de la température, du pH, des gaz.

Le dispositif comprend également une entrée 800 destinée à injecter dans la cuve 100 de la vapeur afin de mettre en oeuvre avec la mise à température de la cuve 100 par l'intermédiaire des chambres annulaires, des cycles de stérilisation à l'intérieure de la cuve 100. Ce même dispositif 800 est en charge de l'injection de gaz sélectionnés pour permettre un développement optimal des microorganismes.

Afin de pouvoir visualiser l'intérieur du dispositif, des hublots sont disposés sur la paroi de la cuve 100.

## Revendications

1. Dispositif destiné à la culture de cellules ou micro-organismes, comprenant une cuve (100) et un couvercle (103) définissant à l'état fermé une enceinte à atmosphère contrôlée, la cuve (100) comprenant une chambre (120) supérieure et une chambre (130) inférieure en communication, le dispositif comprenant un ensemble (200) configuré pour être placé à l'intérieur de la cuve (100) dans la première chambre tout en étant susceptible d'être retiré de celle-ci, l'ensemble (200) comprenant au moins un plateau (201, 202, 203, 204) destiné à recevoir un milieu de culture solide ou liquide,
le dispositif étant **caractérisé en ce que** la chambre (130) inférieure est adaptée pour recevoir un milieu de culture liquide et **en ce que** le dispositif comprend en outre un système (401) de contrôle du débordement du milieu liquide de la chambre inférieure (130) vers la chambre supérieure (120) afin d'éviter un transfert de matière de la chambre (130) inférieure vers la chambre (120) supérieure.

2. Dispositif selon la revendication 1, comprenant en outre un premier système de prélèvement d'un échantillon du milieu solide disposé dans la partie haute de la chambre (120) supérieure, ledit premier système de prélèvement étant configuré pour prélever, de manière stérile, un échantillon du milieu solide sans perturber l'atmosphère régnant dans l'enceinte.

3. Dispositif selon la revendication 2, dans lequel le premier système de prélèvement d'un échantillon comprend une pelle (501), faisant partie d'un système fixe stérilisable in-situ situé en haut de la chambre supérieure (120) qui permet de récupérer le contenu de la pelle sans perturber l'atmosphère régnant dans l'enceinte.

4. Dispositif selon l'une des revendications précédentes, comprenant un deuxième système de prélèvement d'un échantillon du milieu liquide disposé dans la partie basse de la cuve (100) au niveau de la chambre inférieure (130), ledit deuxième système de prélèvement étant configuré pour prélever un échantillon de milieu liquide sans perturber l'atmosphère régnant dans l'enceinte.

5. Dispositif selon l'une des revendications, précédentes dans lequel le système de contrôle du débordement est constitué d'un capteur (401) de niveau du milieu liquide disposé dans la partie supérieure de la chambre inférieure (120) et d'une arrivée de liquide anti-mousse disposée également dans la partie supérieure de la chambre inférieure.

6. Dispositif selon l'une des revendications précédentes, dans lequel la cuve (100) comprend, au niveau de la chambre supérieure (120) et de la chambre inférieure (130) au moins une entrée permettant d'injecter un fluide ou un gaz.

7. Dispositif selon l'une des revendications précédentes, dans lequel la cuve (100) comprend une double enveloppe à l'extérieur permettant, lorsqu'elle est alimentée au moyen d'un fluide ayant une température contrôlée, de stériliser et de contrôler de manière homogène la température interne de l'enceinte.

8. Dispositif selon l'une des revendications précédentes, comprenant un axe central s'étendant depuis le haut de la cuve (100) dans la chambre supérieure 120 et un châssis support, l'ensemble (200) d'au moins un plateau (201, 202, 203, 204) étant porté par ledit châssis support, ledit châssis étant configuré pour être disposé autour de l'axe, l'axe autorisant une rotation des plateaux.

## Patentansprüche

1. Zur Kultivierung von Zellen oder Mikroorganismen bestimmte Vorrichtung, die einen Behälter (100) und einen Deckel (103) umfasst, welche im geschlossenen Zustand ein Gefäß mit kontrollierter Atmosphäre definieren, wobei der Behälter (100) eine obere Kammer (120) und eine untere Kammer (130) umfasst, die miteinander kommunizieren, wobei die Vorrichtung eine Baugruppe (200) umfasst, die so ausgelegt ist, dass sie im Inneren des Behälters (100) in die erste Kammer eingesetzt werden kann, aber dennoch aus dieser herausgenommen werden kann, wobei die Baugruppe (200) mindestens eine Schale (201, 202, 203, 204) umfasst, die dazu bestimmt ist, ein festes oder flüssiges Kulturmedium aufzunehmen,
wobei die Vorrichtung **dadurch gekennzeichnet ist, dass** die untere Kammer (130) geeignet ist, ein flüssiges Kulturmedium aufzunehmen, und dadurch, dass die Vorrichtung weiter ein System (401) zur Kontrolle des Überlaufs des flüssigen Mediums aus der unteren Kammer (130) in die obere Kammer (120) umfasst, um eine Übertragung von Material aus der unteren Kammer (130) in die obere Kammer (120) zu verhindern.

2. Vorrichtung nach Anspruch 1, die weiter ein erstes System zur Entnahme einer Probe des festen Mediums umfasst, das im oberen Teil der oberen Kammer (120) angeordnet ist, wobei das erste Entnahmesystem so ausgelegt ist, dass es in steriler Art und Weise eine Probe des festen Mediums entnehmen kann, ohne die in dem Gefäß herrschende Atmosphäre zu beeinträchtigen.

3. Vorrichtung nach Anspruch 2, wobei das erste System zur Entnahme einer Probe eine Schaufel (501) umfasst, die zu einem oben in der oberen Kammer (120) befindlichen festen, in situ sterilisierbaren System gehört, welches es ermöglicht, den Inhalt der Schaufel zu entnehmen, ohne die in dem Gefäß herrschende Atmosphäre zu beeinträchtigen.

4. Vorrichtung nach einem der vorstehenden Ansprüche, die ein zweites System zur Entnahme einer Probe des flüssigen Mediums umfasst, das im unteren Teil des Behälters (100) im Bereich der unteren Kammer (130) angeordnet ist, wobei das zweite Entnahmesystem so ausgelegt ist, dass es eine Probe von flüssigem Medium entnehmen kann, ohne die in dem Gefäß herrschende Atmosphäre zu beeinträchtigen.

5. Vorrichtung nach einem der vorstehenden Ansprüche, wobei das System zur Kontrolle des Überlaufs aus einem Pegelsensor (401) für das flüssige Medium, der im oberen Teil der unteren Kammer (120) angeordnet ist, und einem Zulauf für Antischaumflüssigkeit besteht, der ebenfalls im oberen Teil der unteren Kammer angeordnet ist.

6. Vorrichtung nach einem der vorstehenden Ansprüche, wobei der Behälter (100) im Bereich der oberen Kammer (120) und der unteren Kammer (130) mindestens einen Einlass umfasst, der es ermöglicht, ein Fluid oder ein Gas zu injizieren.

7. Vorrichtung nach einem der vorstehenden Ansprüche, wobei der Behälter (100) an der Außenseite eine Doppelwand umfasst, die es, wenn sie mit einem Fluid gespeist wird, das eine kontrollierte Temperatur aufweist, ermöglicht, das Gefäß zu sterilisieren und dessen Innentemperatur in homogener Art und Weise zu kontrollieren.

8. Vorrichtung nach einem der vorstehenden Ansprüche, die eine Mittelachse, welche sich von der Oberseite des Behälters (100) in die obere Kammer 120 erstreckt, und einen Tragrahmen umfasst, wobei die Baugruppe (200) aus mindestens einer Schale (201, 202, 203, 204) von dem Tragrahmen getragen wird, wobei der Rahmen so ausgelegt ist, dass er um die Achse herum angeordnet werden kann, wobei die Achse ein Drehen der Schalen gestattet.

## Claims

1. Device for cultivating cells or microorganisms, comprising a vessel (100) and a cover (103) defining in the closed state a controlled-atmosphere enclosure, the vessel (100) comprising an upper chamber (120) and a lower chamber (130) which are connected to one another, the device comprising an assembly (200) designed to be placed inside the vessel (100) in the first chamber and to be removable therefrom, the assembly (200) comprising at least one tray (201, 202, 203, 204) for receiving a solid or liquid culture medium,
the device being **characterised in that** the lower chamber (130) is suitable for receiving a liquid culture medium and **in that** the device further comprises a system (401) for controlling the overflow of the liquid medium from the lower chamber (130) to the upper chamber (120) in order to prevent matter from being transferred from the lower chamber (130) to the upper chamber (120).

2. Device according to claim 1, further comprising a first system for taking a sample of the solid medium arranged in the top portion of the upper chamber (120), said first system for taking being configured to take, in a sterile manner, a sample of the solid medium without disturbing the atmosphere prevailing in the enclosure.

3. Device according to claim 2, wherein the first system for taking a sample comprises a scoop (501), being part of an in-situ sterilisable fixed system located at the top of the upper chamber (120) which makes it possible to recover the contents of the scoop without disturbing the atmosphere prevailing in the enclosure.

4. Device according to one of the preceding claims, comprising a second system for taking a sample of the liquid medium arranged in the bottom portion of the vessel (100) at the lower chamber (130), said second system for taking a sample being configured to take a sample of liquid medium without disturbing the atmosphere prevailing in the enclosure.

5. Device according to one of the preceding claims wherein the system for controlling the overflow is comprised of a level sensor (401) of the liquid medium arranged in the upper portion of the lower chamber (120) and of an antifoam liquid inlet also arranged in the upper portion of the lower chamber.

6. Device according to one of the preceding claims, wherein the vessel (100) comprises, at the upper chamber (120) and the lower chamber (130) at least one inlet making it possible to inject a fluid or a gas.

7. Device according to one of the preceding claims, wherein the vessel (100) comprises a double casing at the outside making it possible, when it is supplied by means of a fluid that has a controlled temperature, to homogeneously sterilise and control the internal temperature of the enclosure.

8. Device according to one of the preceding claims, comprising a central axis extending from the top of the vessel (100) in the upper chamber 120 and a support frame, the assembly (200) of at least one tray (201, 202, 203, 204) being carried by said support frame, said frame being configured to be arranged around the axis, the axis authorising a rotation of the trays.
